# EUROPÄISCHE PATENTSCHRIFT

(11) **EP 0 873 342 B1**
(45) Veröffentlichungstag und Bekanntmachung des Hinweises auf die Patenterteilung: **04.06.2003**
(21) Anmeldenummer: 96943077.6
(22) Anmeldetag: 11.12.1996
(51) Int. Cl.: C07D 493/06, C09B 5/62, C07D 417/06

(54) **1,7-DIAROXY- ODER 1,7-ARYLTHIOSUBSTITUIERTE PERYLEN-3,4,9,10-TETRACARBONSÄUREN, DEREN DIANHYDRIDE UND DIIMIDE**
1,7-DIAROXY- OR 1,7-DIARYLTHIO-SUBSTITUTED PERYLENE-3,4,9,10-TETRACARBOXYLIC ACIDS AND THEIR DIANHYDRIDES AND DIIMIDES
ACIDES 1,7-DIAROXY-OU 1,7-DIARYLTHIO-SUBSTITUES PERYLENE-3,4,9,10-TETRACARBOXYLIQUES, LEURS DIANHYDRIDES ET LEURS DIIMIDES

(30) Priorität: 18.12.1995 DE 19547209
(43) Veröffentlichungstag der Anmeldung: 28.10.1998
(73) Patentinhaber: BASF AKTIENGESELLSCHAFT, 67056 Ludwigshafen (DE)
(72) Erfinder: BÖHM, Arno, D-68305 Mannheim (DE); ARMS, Harald, D-67551 Worms (DE); HENNING, Georg, D-67061 Ludwigshafen (DE); BLASCHKA, Peter, D-67069 Ludwigshafen (DE)
(86) Internationale Anmeldenummer: EP9605519
(87) Internationale Veröffentlichungsnummer: WO97022607

(56) Entgegenhaltungen:
- EP-A- 0 055 363
- EP-A- 0 227 980
- WO-A-94/25504
- WO-A-96/22005
- WO-A-96/22331
- DYES AND PIGMENTS, Bd. 11, Nr. 4, 1989, BARKING,ESSEX, Seiten 303-317, XP000084462 SEYBOLD,G. ET AL.: "New Perylene and Violanthrone Dyestuffs for Fluorescent Collectors "
- CHEMICAL ABSTRACTS, vol. 110, no. 3, 16.Januar 1989 Columbus, Ohio, US; abstract no. 23496v, ROGOVIK,V.I. ET AL.: "Chemistry of Perylene. Halo derivatives of 3,4,9,10-perylenetetracarboxylic acid " Seite 513; XP002029290 & ZH.ORG.KHIM., Bd. 24, Nr. 3, 1988, Seiten 333-335,
- CHEMICAL ABSTRACTS, vol. 93, no. 9, 1.September 1980 Columbus, Ohio, US; abstract no. 95045a, ROGOVIK,V.I. ET AL.: "Chemistry of Perylene. Nitration of perylene-3,4,9,10-tetracarboxylic acid" Seite 622; XP002029291 & ZH.ORG.KHIM., Bd. 16, Nr. 4, 1980, Seiten 867-872,
- CHEMICAL ABSTRACTS, vol. 69, no. 6, 9.August 1968 Columbus, Ohio, US; abstract no. 20409t, KARPUKHIN,P.P. ET AL.: "Fiber-reactive vat dyes dervived from the diimide of perylenetetracarboxylic acid " Seite 1932; XP002029292 & SU 206 782 A (KARPUKHIN,P.P. ET AL.) 8.Dezember 1967

## Beschreibung

Die vorliegende Erfindung betrifft neue, 1,7-disubstituierte perylen-3,4,9,10-tetracarbonsäuredianhydride der allgemeinen Formel I und Perylen-3,4,9,10-tetracarbonsäuren der allgemeinen Formel Ia 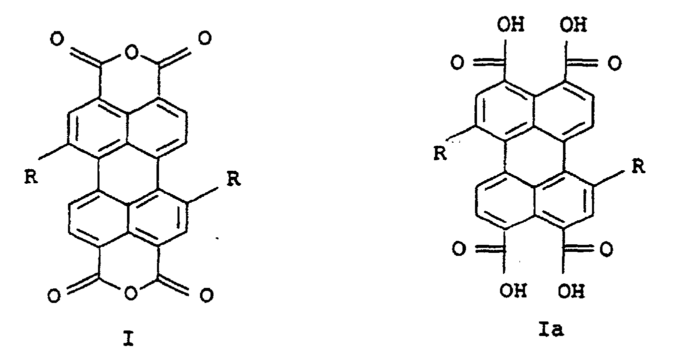 in der
- R: Aryloxy, Arylthio, Hetaryloxy oder Hetarylthio bedeutet, das jeweils durch C₁-C₃₀-Alkyl, dessen Kohlenstoffkette durch eine oder mehrere Gruppierungen -O-, -S-, -NR¹-, -CO- und/oder -SO₂- unterbrochen sein kann und/oder das durch -COOR¹, -SO₃R¹, Hydroxy, Cyano, C₁-C₆-Alkoxy, C₅-C₈-Cycloalkyl oder einen über ein Stickstoffatom gebundenen, 5- bis 7-gliedrigen heterocyclischen Rest, der weitere Heteroatome enthalten kann, ein- oder mehrfach substituiert sein kann, oder durch C₁-C₆-Alkoxy, Cyano, -COOR¹ oder -SO₃R¹ ein- oder mehrfach substituiert sein kann, wobei R¹ für Wasserstoff oder C₁-C₆-Alkyl steht.
sowie ein Verfahren zur Herstellung der Perylen-3,4,9,10-tetracarbonsäuredianhydride (I) bzw. der Säuren (Ia) und deren Verwendung als Pigmente, Laserfarbstoffe und Vorstufen für die Herstellung von Fluoreszenzfarbstoffen, polymeren Farbmitteln, Pigmenten und Pigmentadditiven.

Weiterhin betrifft die Erfindung neue, 1,7-disubstituierte Perylen-3,4,9,10-tetracarbonsäurediimide der allgemeinen Formel VI 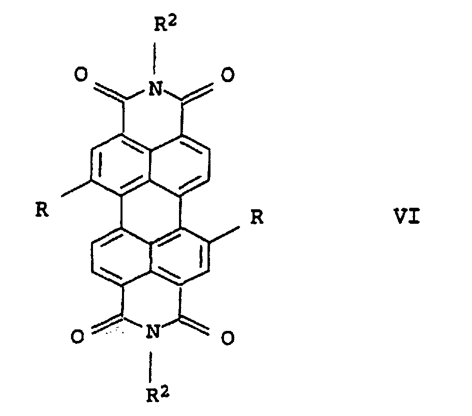 in der die Variablen folgende Bedeutung haben:
- R: Aryloxy, Arylthio, Hetaryloxy oder Hetarylthio, das jeweils durch C₁-C₃₀-Alkyl, dessen Kohlenstoffkette durch eine oder mehrere Gruppierungen -O-, -S-, -NR¹-, -CO- und/oder -SO₂- unterbrochen sein kann und/oder das durch -COOR¹, -SO₃R¹, Hydroxy, Cyano, C₁-C₆-Alkoxy, C₅-C₈-Cycloalkyl oder einen über ein Stickstoffatom gebundenen, 5- bis 7-gliedrigen heterocyclischen Rest, der weitere Heteroatome enthalten kann, einoder mehrfach substituiert sein kann, oder durch C₁-C₆-Alkoxy, Cyano, -COOR¹ oder -SO₃R¹ ein- oder mehrfach substituiert sein kann, wobei R¹ für Wasserstoff oder C₁-C₆-Alkyl steht;
- R²: C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann,
als Zwischenprodukte für die Perylen-3,4,9,10-tetracarbonsäuredianhydride (I) bzw. die Säuren (Ia) sowie ein Verfahren zur Herstellung der Perylen-3,4,9,10-tetracarbonsäurediimide (VI).

Perylen- 3,4,9,10- tetracarbonsauren bzw. deren Anhydride stellen bekanntermaßen wichtige Zwischenprodukte für die Herstellung von Perylimidpigmenten und -farbstoffen dar, eignen sich aber auch selbst zur Einfärbung bzw. Pigmentierung von hochmolekularen organischen Materialien.

Neben der unsubstituierten Perylen-3,4,9,10-tetracarbonsäure, die durch Verseifung von Perylen-3, 4, 9,10-tetracarbonsäurediimid (z.B. aus der EP-A-55 363 bekannt) in konzentrierter Schwefel-säure bei Temperaturen um 200°C erhältlich ist, sind vor allem auch im Perylengerüst substituierte Perylentetracarbonsäuren von Interesse, deren Anwendungseigenschaften wie Löslichkeit, Eigenfarbe und Fluoreszenz durch Einführung geeigneter Substituenten gezielt beeinflußt werden können.

Aus der WO-A-94/25504 sind 1,6,7,12-tetraaroxysubstituierte Perylen-3,4,9,10-tetracarbonsäuredianhydride bekannt, die durch alkalische Verseifung der entsprechenden Diimide in einem polaren, protischen Lösungsmittel hergestellt werden. Die tetraaroxysubstituierten Diimide selbst wurden durch Umsetzung der tetrachlorierten Diimide mit Arylaten erhalten (EP-A-227 980).

1,6,7,12-Tetrasubstituierte Perylen-3,4,9,10-tetracarbonsäurediimide und ihre Anwendung in Elektrolumineszenzanordnungen sind in Dyes and Pigments 11, 303 (1989) bzw. der WO-A-96/22005 beschrieben.

1,7-Disubstituierte Perylen-3,4,9,10-tetracarbonsäuren wie die erfindungsgemäßen Verbindungen (Ia), die wie alle Perylen-3,4,9,10-tetracarbonsäuren in der Regel in Form der Dianhydride vorliegen, sind bislang nicht bekannt. Auch bei den in der EP-A-39 912 und der DRP 412 122 beschriebenen dihalogenierten Perylen-3,4,9,10-tetracarbonsäurediimiden handelt es sich stets um Gemische von verschieden stark halogenierten Produkten (insbesondere von tetra-, tri- und monohalogenierten Produkten), die dihalogenierten Diimide konnten nicht gezielt hergestellt werden. Dies gilt auch für die in Zh. Org. Khim. 24, 635 (1988) und 16, 867 (1980) sowie 28, 1486 (1992) beschriebenen durch Halogen, Nitro, -SO₃H und Hydroxy tetra-bzw. disubstituierten Perylen-3,4,9,10-tetracarbonsäuren. C.A. 69:20409 (1968) erwähnt weiterhin im Perylengerüst durch Hydroxy und Amino substituierte Perylen-3,4,9,10-tetracarbonsäurediimide als Textilfarbstoffe. 1,7-Disubstituierte Perylen-3,4-dicarbonsäureimide werden in der WO-A-96/22331 beschrieben.

Der Erfindung lag daher die Aufgabe zugrunde, neue, disubstituierte Perylen-3,4,9,10-tetracarbonsäuren bzw. -dianhydride bereitzustellen.

Demgemäß wurden die 1,7-disubstituierten Perylen-3,4,9,10-tetracarbonsäuredianhydride und die entsprechenden Säuren der eingangs definierten Formeln I und Ia (im folgenden als "Dianhydride I" bezeichnet) gefunden.

Bevorzugte Dianhydride I sind dem Unteranspruch zu entnehmen.

Weiterhin wurde ein Verfahren zur Herstellung der Dianhydride I gefunden, welches dadurch gekennzeichnet ist, daß man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III

   R² ― NH₂ III

   in der R² C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann, bedeutet, umsetzt,
b) die in Schritt a) gebildeten 1,7-Dibromperylen-3,4,9,10-tetracarbonsäurediimide der allgemeinen Formel IV 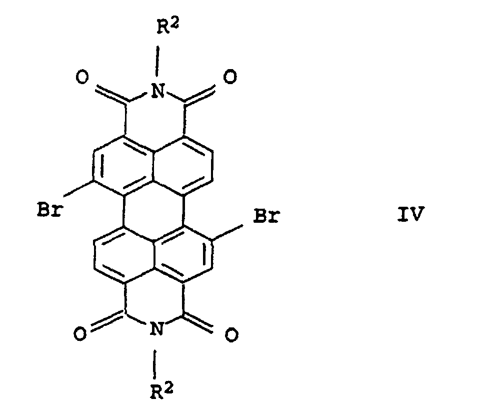 in Gegenwart eines inerten aprotischen Lösungsmittels und einer nicht oder nur schwach nucleophilen Base mit einem aromatischen Alkohol oder Thioalkohol der allgemeinen Formel V

   H―R V

   umsetzt und
c) die in Schritt b) gebildeten, 1,7-disubstituierten Perylen-3,4,9,10-tetracarbonsäurediimide der allgemeinen Formel VI 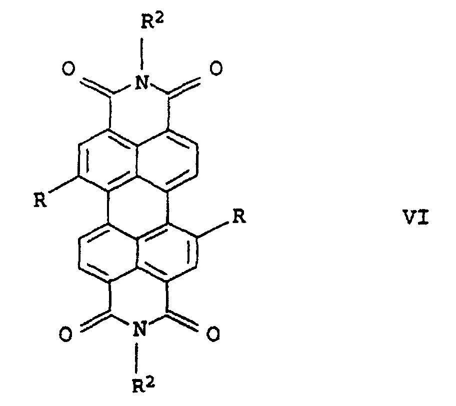 in Gegenwart eines polaren protischen Lösungsmittels und einer Base zu den Dianhydriden I verseift.

Zudem wurden die 1,7-disubstituierten Perylen-3,4,9,10-tetracarbonsäurediimide der eingangs definierten Formel VI (im folgenden als "Perylimide VI" bezeichnet) als Zwischenprodukte für die Dianhydride I sowie Verfahren zur Herstellung der Perylimide VI gefunden, welche die Schritte a) und b) des Verfahrens zur Herstellung der entsprechenden Dianhydride I umfassen.

Bevorzugte Perylimide VI sind dem Unteranspruch zu entnehmen.

Außerdem wurde ein zweites Verfahren zur Herstellung der Dianhydride I gefunden, welches dadurch gekennzeichnet ist, daß man 1,7-Dibromperylen-3,4,9,10-tetracarbonsäuredianhydrid (II) bzw. 1,7-Dibromperylen-3,4,9,10-tetracarbonsäure (IIa) in Gegenwart eines aprotischen Lösungsmittels und einer anorganischen Base mit einem aromatischen Alkohol oder Thioalkohol der Formel V umsetzt.

Schließlich wurde ein Verfahren zur Herstellung von 1,7-Dibromperylen-3,4,9,10-tetracarbonsäuredianhydrid (IIa) bzw. 1,7-Dibromperylen-3,4,9,10-tetracarbonsäure durch Bromierung von Perylen-3,4,9,10-tetracarbonsäuredianhydrid bzw. Perylen-3,4,9,10-tetracarbonsäure in 100 gew.-%iger Schwefelsäure gefunden, welches dadurch gekennzeichnet ist, daß man die Bromierung in Gegenwart von Iod bei einer Temperatur von 80 bis 90°C vornimmt und das Brom langsam zudosiert.

Nicht zuletzt wurde die Verwendung der Dianhydride I als Pigmente, Laserfarbstoffe und Vorstufen für die Herstellung von Fluoreszenzfarbstoffen, polymeren Farbmitteln, Pigmenten und Pigmentadditiven gefunden.

Schließlich wurde auch die Verwendung der Perylimide VI als Pigmente und Farbstoffe zum Einfärben von hochmolekularen organischen und von anorganischen Materialien, als Laserfarbstoffe und organische Materialien für Elektrolumineszenzanwendungen gefunden.

Geeignete unsubstituierte Reste R sind dabei Phenoxy, Phenylthio, 2-Naphthyloxy, 2-Naphthylthio, 2-, 3- und 4-Pyridyloxy, 2-, 3-und 4-Pyridylthio, 2-, 4- und 5-Pyrimidyloxy und 2-, 4- und 5-Pyrimidylthio, wobei Phenoxy und 2-Naphthyloxy bevorzugt sind.

Die als Substituenten für die Reste R auftretenden Alkylgruppen (sowie auch die Alkylreste R¹ und R²) können sowohl geradkettig als auch verzweigt sein.

Als Beispiele für diese Substituenten (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-(N-Methylamino)- und 2-(N-Ethylamino)ethyl, 2-(N,N-Dimethylamino)ethyl, 2- und 3-(N,N-Dimethylamino)propyl, 3-(N-Isopropylaminopropyl, 2- und 4-(N-Propylamino)butyl, 2- und 4-(N,N-Dimethylamino)butyl, 6-Methyl-3,6-diazaheptyl, 3,6-Dimethyl-3,6-diazaheptyl, 3,6-Diazoaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Methylcarboxymethyl, Ethylcarboxymethyl, Propylcarboxymethyl, Butylcarboxymethyl, Pentylcarboxymethyl, Hexylcarboxymethyl, Methyl-2-carboxyethyl, Ethyl-2-carboxyethyl, Propyl-2-carboxyethyl, Butyl-2-carboxyethyl, Pentyl-2-carboxyethyl, Hexyl-2-carboxyethyl, Methyl-3-carboxypropyl, Ethyl-3-carboxypropyl, Propyl-3-carboxypropyl, Butyl-3-carboxypropyl, Pentyl-3-carboxypropyl, Hexyl-3-carboxypropyl, Methyl-4-carboxybutyl, Methyl-5-carboxypentyl, Methyl - 6 - carboxyhexyl, Methyl - 8 - carboxyoctyl, Methyl-10-carboxydecyl, Methyl-12-carboxydodecyl und Methyl-14-carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
5 Methylsulfomethyl, Ethylsulfomethyl, Propylsulfomethyl, Butylsulfomethyl, Pentylsulfomethyl, Hexylsulfomethyl, Methyl-2-sulfoethyl, Ethyl-2-sulfoethyl, Propyl-2-sulfoethyl, Butyl-2-sulfoethyl, Pentyl-2-sulfoethyl, Hexyl-2-sulfoethyl, Methyl-3-sulfopropyl, Ethyl-3 -sulfopropyl, Propyl-3-sulfopropyl, Butyl-3-sulfopropyl, Pentyl-3-sulfopropyl und Hexyl-3-sulfopropyl, Methyl-4-sulfobutyl, Methyl-5-sulfopentyl, methyl-6-sulfohexyl, Methyl - 8 - sulfooctyl, Methyl-10-sulfodecyl, Methyl-12-sulfododecyl und Methyl-14-sulfotetradecyl;
2-Hydroxyethyl, 2- und 3-Hydroxypropyl, 1-Hydroxyprop-2-yl, 2-und 4-Hydroxybutyl, 1-Hydroxybut-2-yl und 8-Hydroxy-4-oxaoctyl, 2-Cyanoethyl, 3-Cyanopropyl, 2-Methyl-3-ethyl-3-cyanopropyl, 7-Cyano-7-ethylheptyl und 4-Methyl-7-methyl-7-cyanoheptyl;
Methoxy, Ethoxy, Propoxy, Isopropoxy, Butoxy, Isobutoxy, tert.-Butoxy, Pentoxy, Isopentoxy, Neopentoxy, tert.-Pentoxy und Hexoxy;
Cyclopentyl, Cyclohexyl, Cycloheptyl, Cyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl.

Beispiele für geeignete Reste R¹ sind neben Wasserstoff die obengenannten C₁-C₆-Alkylreste.

Als Beispiele für geeignete Reste R² sind die oben aufgeführten C₄-C₃₀-Alkylreste, die durch -O-, -S- oder -CO- unterbrochenen C₄-C₃₀-Alkylreste und die C₅-C₈-Cycloalkylreste sowie Arylreste, z.B. Naphthyl und insbesondere Phenyl, zu nennen, die durch 1, 2 oder 3 der genannten C₁-C₆-Alkyl- oder -Alkoxyreste substituiert sein können, wie

2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl, 2-, 3- und 4-Isobutylphenyl, 2,4-, 3,5 und 2,6-Diisobutylphenyl, 2,4,6-Triisobutylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl.

Die Herstellung der Dianhydride I kann nach dem erfindungsgemäßem mehrstufigen Verfahren, bei dem in Schritt a) 1,7-Dibromperylen-3,4,9.10-tetracarbonsäuredianhydrid (II) mit einem primären Amin III zu dem entsprechenden 1.7-Dibromperylen-3,4,9,10-tetracarbonsäurediimid IV umgesetzt wird, welches in Schritt b) mit einem aromatischen Alkohol oder Thioalkohol V zum Perylimid VI umgesetzt wird, das schließlich in Schritt c) basisch zum Dianhydrid I verseift wird, oder nach dem erfindungsgemäßen einstufigen Verfahren durch direkte Umsetzung von 1,7-Dibromperylen-3,4,9,10-tetracarbonsäuredianhydrid (II) mit dem aromatischen Alkohol oder Thioalkohol V erfolgen.

In der Regel sind die bei der einstufigen Verfahrensvariante anfallenden Produkte jedoch stärker verunreinigt und müssen daher noch einem Nachreinigungsschritt, z.B. einer fraktionierten Kristallisation oder einer Säulenfiltration über Kieselgel unterzogen werden, weshalb in den meisten Fällen die dreistufige Verfahrensvariante vorzuziehen ist.

Das als Ausgangsprodukt für das erfindungsgemäße Herstellungsverfahren dienende 1,7-Dibromperylen-3,4,9,10-tetracarbonsäuredianhydrid (II) ist erfindungsgemäß durch selektive Bromierung von Perylen-3,4,9,10-tetracarbonsäuredianhydrid in 100 gew.-%iger Schwefelsäure (Monohydrat) zu erhalten.

Zweckmäßigerweise geht man dabei so vor, daß man Perylen-3,4,9,10-tetracarbonsäuredianhydrid zunächst 2 bis 16 h in der Schwefelsäure rührt und diese Mischung anschließend nach Zugabe eines Halogenierungskatalysators wie Iod (bevorzugt 30 bis 40 mmol je mol Anhydrid) auf die Reaktionstemperatur (in der Regel 80 bis 90°C) erhitzt. Dann tropft man das Brom langsam (üblicherweise in 6 bis 10 h) zu, wobei bevorzugt 2 bis 2,5 mol Brom (Br₂) je mol Anhydrid verwendet werden. Nach Abkühlen auf Raumtemperatur und Verdrängen des nicht umgesetzten Broms durch Stickstoff erniedrigt man die Schwefelsäurekonzentration durch portionsweise Zugabe von Wasser auf etwa 85 bis 88 Gew.-%.

Die Aufarbeitung des Reaktionsgemisches auf das 1,7-Dibromperylen-3,4,9,10-tetracarbonsäuredianhydrid (II) kann dann durch Abfiltrieren des ausgefallenen Produkts, Waschen mit 85 bis 88 gew.-%iger Schwefelsäure, Einrühren in Wasser, erneutes Abfiltrieren, Waschen mit Wasser und anschließendes Trocknen erfolgen.

Schritt a) des erfindungsgemäßen dreistufigen Herstellungsverfah-rens, die Umsetzung des 1,7-Dibromperylen-3,4,9,10-tetracarbonsäuredianhydrids (II) mit dem primären Amin, wird in Gegenwart eines polaren aprotischen Lösungsmittels und gegebenenfalls eines Imidierungskatalysators vorgenommen.

Als polare aprotische Lösungsmittel eignen sich dabei vor allem aprotische Stickstoffheterocyclen wie Pyridin, Pyrimidin, Chinolin, Isochinolin, Chinaldin, N-Methylpiperidin, N-Methylpiperidon und insbesondere N-Methylpyrrolidon.

Die Menge an Lösungsmittel ist an sich nicht kritisch, üblicherweise kommen 5 bis 20 kg, vorzugsweise 10 bis 15 kg, Lösungsmittel je kg (II) zum Einsatz.

Als Imidierungskatalysatoren eignen sich organische und anorganische Säuren, z.B. Ameisensäure, Essigsäure, Propionsäure und Phosphorsäure, die bevorzugt in möglichst konzentrierter Form eingesetzt werden, sowie organische und anorganische Salze von Übergangsmetallen wie Zink, Eisen und Kupfer und von Magnesium, z.B. Zinkacetat, Zinkpropionat, Zinkoxid, Eisen(II)acetat, Eisen (III) chlorid, Eisen (II) sulfat, Kupfer (II) acetat, Kupfer (II)-oxid und Magnesiumacetat. Selbstverständlich kann man auch Mischungen der genannten Katalysatoren verwenden.

Die Anwesenheit eines Imidierungskatalysators empfiehlt sich insbesondere bei der Umsetzung aromatischer Amine und ist auch bei der Umsetzung cycloaliphatischer Amine vorteilhaft, während bei der Umsetzung vor allem kurzkettiger aliphatischer Amine üblicherweise kein Katalysator erforderlich ist.

In der Regel werden 5 bis 80 Gew.-% Katalysator, bezogen auf (II), eingesetzt. Bevorzugte Mengen betragen bei den organischen Säuren 50 bis 80 Gew.-% und bei den Übergangsmetall- und Magnesiumsalzen 10 bis 40 Gew.-%, jeweils bezogen auf (II).

Als primäre Amine können bei dem erfindungsgemäßen Herstellungsverfahren alle unter den Reaktionsbedingungen stabilen Amine eingesetzt werden, die mit Perylen-3,4,9,10-tetracarbonsäuredianhydriden Diimide bilden, die unter basischen Bedingungen verseift werden können.

Beispiele für besonders bevorzugte primäre Amine III sind Stearylamin, 5-Nonylamin, Cyclopentylamin, Cyclohexylamin, Cycloheptylamin, Anilin, 4-Methylanilin, 4-Ethylanilin, 4-tert.-Butylanilin, 3,5-Dimethylanilin, 3,5-Diethylanilin und 3,5-Di-tert.butylanilin.

Üblicherweise liegt das Molverhältnis von Amin III zu (II) bei etwa 2:1 bis 4:1, vorzugsweise bei etwa 2,2:1 bis 3:1.

Die Reaktionstemperatur beträgt in Schritt a) im allgemeinen 40 bis 180°C. Bei der Umsetzung aliphatischer und cycloaliphatischer Amine sind Temperaturen von 60 bis 100°C und bei der Umsetzung aromatischer Amine Temperaturen von 120 bis 160°C bevorzugt.

Es empfiehlt sich, unter Verwendung einer Schutzgasatmosphäre (z.B. Stickstoff) zu arbeiten.

Man kann Schritt a) des erfindungsgemäßen Verfahrens bei Normaldruck oder bei einem Überdruck von üblicherweise bis zu 10 bar durchführen. Die Arbeitsweise unter Druck ist vor allem beim Einsatz flüchtiger Amine (Siedepunkt ≤ etwa 180°C) zweckmäßig.

Üblicherweise ist die Umsetzung in 2 bis 10 h, vor allem in 4 bis 7 h, beendet.

Verfahrenstechnisch geht man in Schritt a) zweckmäßigerweise wie folgt vor:
Man legt 1,7-Dibromperylen-3,4,9,10-tetracarbonsäuredianhydrid (II), Lösungsmittel und gegebenenfalls Katalysator vor, fügt das Amin III unter Rühren bei Raumtemperatur zu, spült die Apparatur etwa 15 min mit Stickstoff, erhitzt das Gemisch unter Rühren auf die Reaktionstemperatur und hält es etwa 4 bis 7 h bei dieser Temperatur. Nach dem Abkühlen auf Raumtemperatur wird das Reaktionsprodukt abfiltriert, mit einem aliphatischen Alkohol wie Methanol gewaschen und getrocknet.
Soll die Umsetzung unter Druck vorgenommen werden, so verwendet man eine Druckapparatur als Reaktionsgefäß, auf die man nach dem Einfüllen der Komponenten einen Stickstoffdruck von etwa 1 bis 2 bar gibt, erhitzt anschließend die gewünschte Zeit auf die Reaktionstemperatur und entspannt nach dem Abkühlen.
In der Regel hat das in Schritt a) erhaltene 1,7-Dibromperylen-3,4,9,10-tetracarbonsäurediimid IV bereits einen so hoben Wertgehalt (>95 %), daß es direkt für die Folgeumsetzungen verwendet werden kann. Analysenreine Produkte mit Reinheiten von >98 % können durch Lösen in einem halogenierten Kohlenwasserstoff wie Methylenchlorid, Chloroform oder Tetrachlorethan, Filtration über Kieselgel und Einengen des Filtrats zur Trockene hergestellt werden.

Schritt b) des erfindungsgemäßen dreistufigen Herstellungsverfahrens, die Umsetzung des 1,7-Dibromperylen-3,4,9,10-tetracarbonsäurediimids IV mit dem aromatischen Alkohol oder Thioalkohol V, wird in Gegenwart eines inerten aprotischen Lösungsmittels und einer nicht oder nur schwach nucleophilen Base vorgenommen.

Als inerte aprotische Lösungsmittel eignen sich dabei vor allem Stickstoffheterocyclen wie Pyridin, Pyrimidin, Chinolin, Isochinolin, Chinaldin und insbesondere N-Methylpyrrolidon.

Die Menge an Lösungsmittel ist an sich nicht kritisch, üblicherweise kommen 10 bis 50 kg, vorzugsweise 25 bis 35 kg, Lösungsmittel je kg Diimid IV zum Einsatz.

Als Basen eignen sich besonders Alkalimetallhydroxide, z.B. Natriumhydroxid und Kaliumhydroxid, und vor allem Alkalimetallcarbonate, z.B. Natriumcarbonat und Kaliumcarbonat, die in wasserfreier Form verwendet werden.

In der Regel werden 2 bis 3, vorzugsweise 2,2 bis 2,5, Moläquivalente Base je mol Diimid eingesetzt.

Die Reaktionstemperatur beträgt in Schritt b) üblicherweise 60 bis 180°C, vor allem 80 bis 140°C.

Es empfiehlt sich, unter Verwendung einer Schutzgasatmosphäre (z.B. Stickstoff) zu arbeiten.

Das Molverhältnis von aromatischem Alkohol oder Thioalkohol V zu Diimid IV liegt in der Regel bei 2:1 bis 3:1, vorzugsweise bei 2,0:1 bis 2,2:1.

Üblicherweise ist die Umsetzung zu den Perylimiden VI nach 1 bis 5 h, vor allem 1 bis 2 h, beendet.

Verfahrenstechnisch geht man in Schritt b) zweckmäßigerweise wie folgt vor:
Man legt eine gerührte Suspension von Diimid IV, Alkohol oder Thioalkohol V und Base im Lösungsmittel vor und erhitzt unter Stickstoff 1 bis 2 h auf die Reaktionstemperatur. Nach dem Abkühlen auf Raumtemperatur trägt man das Reaktionsgemisch in das etwa 3fache Volumen an einer verdünnten anorganischen Säure, z.B. 5 bis 10 gew.-%iger Salzsäure ein, filtriert das ausgefallene Reaktionsprodukt ab, wäscht mit Wasser bis zum neutralen Ablauf und trocknet im Vakuum.

In der Regel haben die so behandelten Perylimide VI bereits einen so hohen Wertgehalt (>95%), daß auf eine weitere Reinigung verzichtet werden kann. Analysenreine Proben können durch Umkristallisation aus halogenierten Kohlenwasserstoffen wie Methylenchlorid oder Chloroform oder durch Filtration einer Lösung in diesen Lösungsmitteln über Kieselgel und anschließendes Einengen gewonnen werden.

Schritt c) des erfindungsgemäßen dreistufigen Herstellungsverfahrens, die Verseifung des Perylimids VI zum Dianhydrid I, wird in Gegenwart eines polaren protischen Lösungsmittels und einer Base vorgenommen.

Als polare protische Lösungsmittel kommen dabei insbesondere C₁-C₁₀-Alkanole wie Ethanol, Propanol, n-Butanol, tert.-Butanol, 2-Methyl-2-butanol, n-Hexanol, n-Decanol und vorzugsweise Isopropanol in Frage. Zweckmäßigerweise wird zur Beschleunigung der Verseifungsreaktion noch Wasser, im allgemeinen 0,1 bis 0.2 mol je mmol Perylimid VI, zugesetzt.

Die Menge an Lösungsmittel ist an sich nicht kritisch, üblicherweise werden 50 bis 200 kg, bevorzugt 60 bis 80 kg, je kg Perylimid VI eingesetzt.

Als Base eignen sich vor allem Alkalimetallhydroxide wie Natriumhydroxid und Kaliumhydroxid, die in der Regel in Mengen von 4 bis 10 kg, vorzugsweise 5 bis 7 kg, je kg Perylimid VI zum Einsatz kommen.

Die Reaktionstemperatur beträgt in Schritt c) üblicherweise 20 bis 140°C, insbesondere 40 bis 90°C.

Im allgemeinen ist die Verseifung in 3 bis 10 h, vor allem 4 bis 6 h, beendet.

Verfahrenstechnisch geht man in Schritt c) zweckmäßigerweise wie folgt vor:
Man erhitzt eine Mischung von Perylimid VI, Lösungsmittel und Base für 4 bis 6 h auf die gewünschte Reaktionstemperatur, filtriert das nach Abkühlen auf Raumtemperatur ausgefallene Rohprodukt ab und wäscht es mit einem Alkohol wie Isopropanol oder propanol bis zum farblosen Ablauf. Zur weiteren Reinigung trägt man das erhaltene Dianhydrid I zweckmäßigerweise in die 30- bis 100-fache Menge verdünnter anorganischer Säure, z.B. 5 bis 12 gew.-%iger Salzsäure, ein, kocht kurz auf, filtriert nach dem Abkühlen ab, wäscht mit Wasser neutral und trocknet.

Bei der erfindungsgemäßen einstufigen Verfahrensvariante wird das 1,7-Dibromperylen-3,4,9,10-tetracarbonsäuredianhydrid (II) direkt mit dem aromatischen Alkohol oder Thioalkohol V umgesetzt.

Die Reaktionsbedingungen, wie die Art und die Menge des Lösungsmittels und der Base sowie die Reaktionstemperatur, entsprechen denjenigen bei Schritt b) des dreistufigen Verfahrens. Jedoch beträgt das Molverhältnis von V zu (II) in der Regel 2:1 bis 10:1, vorzugsweise 4:1 bis 6:1. Auch werden im allgemeinen längere Reaktionszeiten von 4 bis 10 h, insbesondere 5 bis 7 h, benötigt.

Verfahrenstechnisch geht man bei der einstufigen Variante zweckmäßigerweise wie folgt vor:

Man legt eine gerührte Suspension von 1,7-Dibromperylen-3,4,9,10-tetracarbonsäuredianhydrid (II), Alkohol oder Thioalkohol V und Base im Lösungsmittel vor und erhitzt unter Stickstoff 5 bis 7 h auf die Reaktionstemperatur. Nach dem Abkühlen auf etwa 50°C fil-triert man das ausgefallene Reaktionsprodukt ab und wäscht mit kaltem Lösungsmittel. Dann rührt man den Feststoff in verdünnter anorganischer Säure, z.B. 5 bis 10 gew.-%iger Salzsäure, auf, dekantiert anschließend die flüssige Phase ab, füllt mit heißem Wasser auf, rührt etwa 30 min bei 80 bis 85°C, filtriert vorzugsweise heiß ab, wäscht mit heißem Wasser bis zum neutralen Ablauf und trocknet im Vakuum.

Die so erhaltenen Produkte sind in der Regel zu etwa 10 bis 15 % mit nicht oder nur teilweise umgesetztem Ausgangsmaterial (II) verunreinigt, weshalb eine Nachreinigung, z.B. durch fraktionierte Kristallisation in einem geeigneten, höhersiedenden Lösungsmittel wie N-Methylpyrrolidon oder Tetrachlorethan oder durch eine Filtration einer Lösung des Rohproduktes in diesen Lösungsmitteln über eine kurze Kieselgelsäule zu empfehlen ist.

Mit Hilfe der erfindungsgemäßen Herstellungsverfahren (einschließlich Nachreinigung bei der einstufigen Variante) können die 1,7-disubstituierten Perylen-3,4,9,10-tetracarbonsäuredianhydride I auf verfahrenstechnisch einfache, wirtschaftliche Weise in hohen Reinheiten (im allgemeinen > 95 %) und guten Ausbeuten (im allgemeinen > 70 % beim dreistufigen und >55 % beim einstufigen Verfahren) erhalten werden.

Die erfindungsgemäßen Dianhydride I eignen sich vorteilhaft zur Pigmentierung von Druckfarben, Anstrichmitteln, insbesondere Tagesleuchtfarben und Kunststoffen, als Laserfarbstoffe und als Vorstufen für die Herstellung von Fluoreszenzfarbstoffen, polymeren Farbmitteln, Pigmenten und Pigmentadditiven.

Auch die erfindungsgemäßen Perylimide VI können vorteilhaft als Pigmente und Farbstoffe zum Einfärben von hochmolekularen organischen und von anorganischen Materialien, als Laserfarbstoffe und organische Materialien für Elektrolumineszenzanwendungen verwendet werden.

### Beispiele

### A) Herstellung von 1,7-Dibromperylen-3,4,9,10-tetracarbonsäuredianhydrid (II)

### Beispiel 1

Eine Mischung von 292,5 g (0,75 mol) Perylen-3,4,9,10-tetracarbonsäuredianhydrid (Wertgehalt > 98 %) und 4420 g 100 gew.-%iger Schwefelsäure wurde nach 12stündigem Rühren bei Raumtemperatur und anschließender Zugabe von 7 g Iod auf 85°C erhitzt. Dann wurden 262,5 g (1,64 mol) Brom in 8 h zugetropft.

Nach Abkühlen auf Raumtemperatur und Verdrängen des überschüssigen Broms durch Stickstoff wurde die Schwefelsäurekonzentration des Reaktionsgemisches durch portionsweise Zugabe von insgesamt 670 g Wasser in 1 h auf 86 Gew.-% erniedrigt. Nach dem Abkühlen des sich dabei auf 85°C erhitzenden Reaktionsgemisches auf Raumtemperatur wurde das ausgefallene Produkt über eine G4-Glasfritte abfiltriert, mit 3 kg 86 gew.-%iger Schwefelsäure gewaschen, dann in 5 l Wasser aufgerührt, erneut abfiltriert, neutral gewaschen und bei 120°C im Vakuum getrocknet.

Es wurden 370 g II in Form eines leuchtendroten, feinkristallinen Pulvers mit einem Schmelzpunkt > 360°C und einem Wertgehalt von >98 % erhalten, was einer Ausbeute von 90 % entspricht.

Analytische Daten:
Elementaranalyse (Gew.-% ber./gef.):
C: 52,4/52,1; H: 1,1/1.1; O: 17,45/17,4; Br: 29,1/29,4; IR (KBr) : ν = 1782+1770 (s, C=O), 1735+1723 (s, C=O) cm⁻¹; UV/VIS (H₂SO₄): *λ*ₘₐₓ (ε) = 408 (10309), 520 (29410), 554 (43141) nm.

### B1) Herstellung von 1,7-diaroxysubstituierten Perylen-3,4,9,10-tetracarbonsäuredianhydriden I nach der dreistufigen Verfahrensvariante

### a) Herstellung von 1,7-Dibromperylen-3,4,9,10-tetracarbonsäurediimiden (IV)

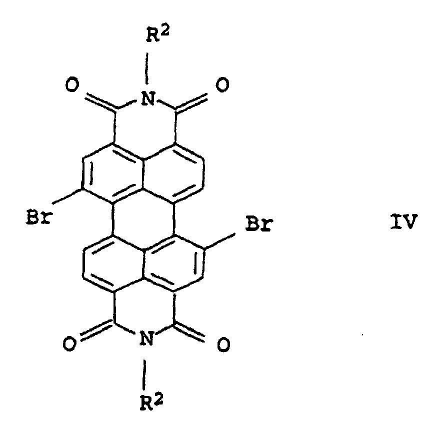

### Beispiele 2 bis 4

Zu einer Mischung von 69,9 g (127 mmol) 1,7-Dibromperylen-3,4,9,10-tetracarbonsäuredianhydrid (II) aus Beispiel 1 in 900 ml N-Methyl-2-pyrrolidon wurden unter Rühren zunächst a g Imidierungskatalysator und dann portionsweise insgesamt 381 mmol (280 mmol in Beispiel 3) des Amins R₂-NH₂ (III) zugegeben. Anschließend wurde das Reaktionsgemisch unter Stickstoff auf T°C erhitzt und 6 h bei dieser Temperatur gerührt.

Nach dem Abkühlen auf Raumtemperatur wurde das ausgefallene Reaktionsprodukt abfiltriert, mit insgesamt 2 l Methanol gewaschen und bei 100°C im Vakuum getrocknet.

Weitere Einzelheiten zu diesen Versuchen sowie deren Ergebnisse sind in Tabelle 1 zusammengestellt.

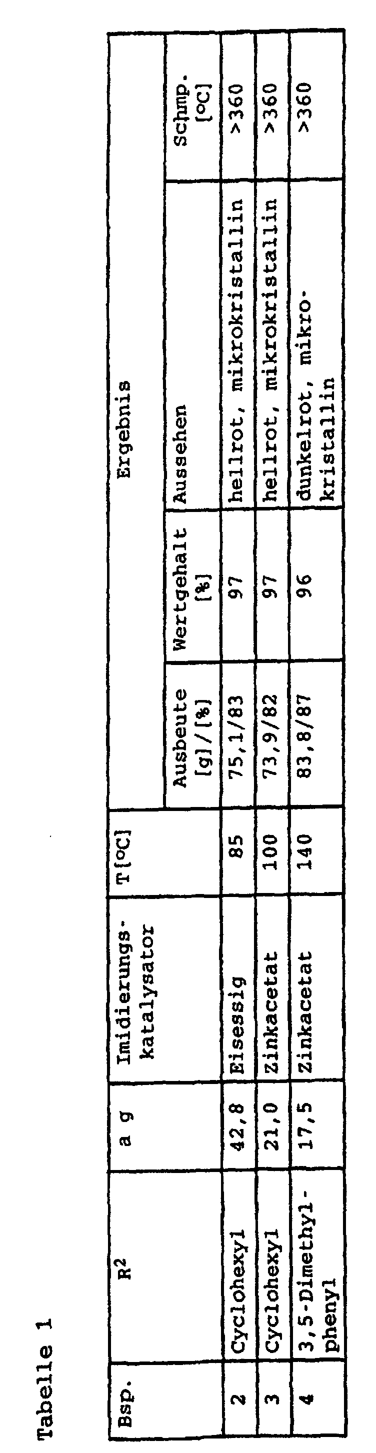

Analytische Daten zu Beispiel 2 und 3:
Elementaranalyse (Gew.-% ber./gef.):
C: 60,7/60,6; H: 4,0/4,0; N: 3,9/3,9; O: 9,0/9,0;
Br: 22,4/22,3;
IR (KBr): ν = 1698 (s, C=O), 1655 (s, C=O) cm⁻¹;
UV/VIS (CHCl₃): λₘₐₓ (ε) = 491 (33411), 526 (50033) nm.

Analytische Daten zu Beispiel 4:
Elementaranalyse (Gew.-% ber./gef.):
C: 63,5/63,2; H: 3,2/3,25; N: 3,7/3,7; O: 8,5/8,7;
Br: 21,1/21,0;
Masse (FD): m/z = 754 (M⁺, 100 %);
IR (KBr): ν = 1696 (s, C=O), 1653 (s, C=O cm⁻¹;
UV/VIS (CHCl₃): λₘₐₓ (ε) = 485 (28570), 532 (44201) nm.

### b) Herstellung von 1,7-diaroxysubstituierten Perylen-3,4,9,10-tetracarbonsäurediimiden VI

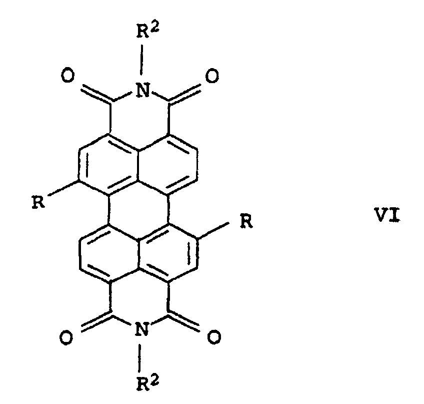

### Beispiele 5 bis 7

Jeweils 20 mmol des 1,7-Dibromperylen-3,4,9,10-tetracarbonsäurediimids (IV) aus Beispiel 2 bzw. 4 wurden unter Rühren in 450 ml N-Methylpyrrolidon eingetragen, nacheinander mit 6,4 g (46 mmol) wasserfreiem Kaliumcarbonat und a g (40 mmol) des Hydroxyaromaten V versetzt und unter Stickstoff 1,5 h auf 120°C erhitzt.

Nach dem Abkühlen auf Raumtemperatur wurde die Reaktionsmischung unter Rühren in 1,5 l einer 6 gew.-%igen Salzsäure eingetragen. Das ausgefallene Reaktionsprodukt wurde abfiltriert, mit Wasser neutral gewaschen und bei 100°C im Vakuum getrocknet.

Weitere Einzelheiten zu diesen Versuchen sowie deren Ergebnisse sind in Tabelle 2 zusammengestellt.

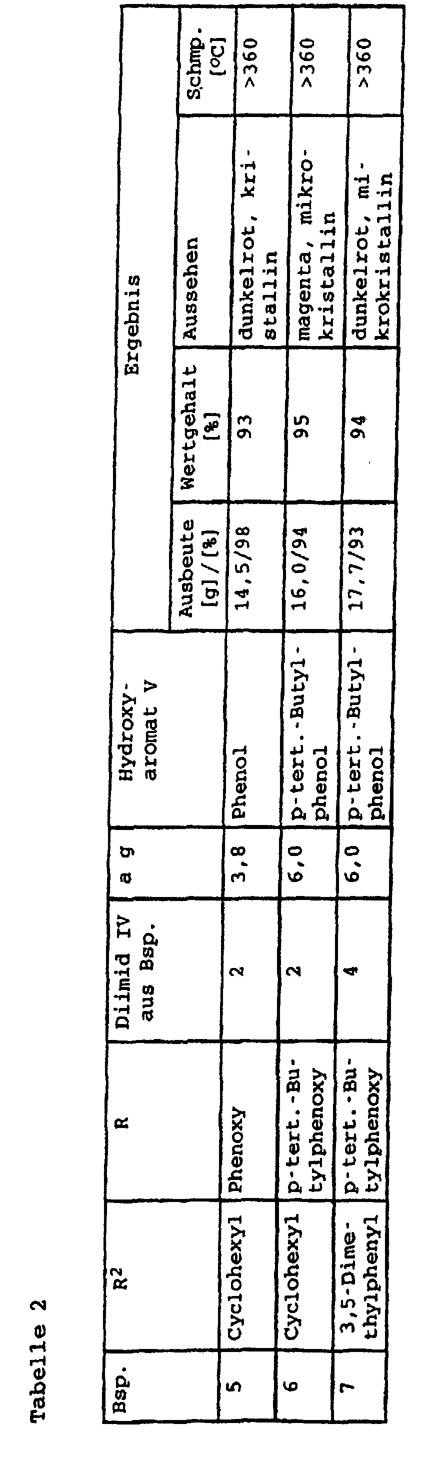

Analytische Daten zu Beispiel 5:
Elementaranalyse (Gew.-% ber./gef.):
C: 78,0/77,5; H: 5,2/5,3; N: 3,8/3,7; O: 13,0/13,4;
Masse (FD) : m/z = 738 (M⁺, 100 %);
IR (KBr): ν = 1695 (s, C=O), 1654 (s, C=O) cm⁻¹;
UV/VIS (CHCl₃): λₘₐₓ (ε) = 401 (7455), 513 (37102), 549 (55004) nm.

Analytische Daten zu Beispiel 6:
Elementaranalyse (Gew.-% ber./gef.):
C: 79,0/78,8; H: 6,4/6,4; N: 3,3/3,2; O: 11,3/11,4;
Masse (FD): m/z = 850 (M⁺, 100 %);
IR (KBr): ν = 1697 (s, C=O), 1654 (s, C=O) cm⁻¹;
UV/VIS (CHCl₃): λₘₐₓ (ε) = 404 (9447), 512 (34785),
547 (52117) nm.

Analytische Daten zu Beispiel 7:
Elementaranalyse (Gew.-% ber./gef.);
C: 80,5/80,4; H: 5,6/5,6; N: 3,1/3,1; O: 10,7/10,8;
Masse (FD) : m/z = 894 (M⁺, 100 %);
IR (KBr): ν = 1699 (s, C=O), 1651 (s, C=O) cm⁻¹;
UV/VIS (CHCl₃): λₘₐₓ (ε) = 408 (10703), 511 (32187), 544 (58330) nm.

### c) Herstellung von 1,7-diaroxysubstituierten Perylen-3,4,9,10-tetracarbonsäuredianhydriden I

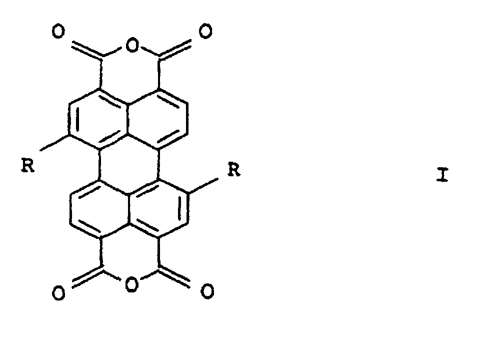

### Beispiele 8 und 10

Eine Mischung von jeweils 10 g des 1,7-Diaroxyperylen-3,4,9,10-tetracarbonsäurediimids (VI) aus Beispiel 5, 6 bzw. 7, 11 Isopropanol, 65 g Kaliumhydroxid und 26 g Wasser wurde 5 h unter RückfluB erhitzt.

Nach dem Abkühlen auf Raumtemperatur wurde das ausgefallene Reaktionsprodukt abfiltriert, mit Isopropanol bis zum farblosen Ablauf gewaschen, dann unter Rühren in 1 l 10 gew.-%ige Salzsäure eingetragen und kurz zum Sieden erhitzt. Nach Abkühlen auf Raumtemperatur wurde das Produkt erneut abfiltriert, mit Wasser neutral gewaschen und bei 100°C im Vakuum getrocknet.

Weitere Einzelheiten zu diesen Versuchen sowie deren Ergebnisse sind in Tabelle 3 zusammengestellt. Die Bestimmung des Wertgehaltes der Produkte erfolgte durch UV/VIS-Spektroskopie und halbquantitative Dünnschichtchromatographie an Kieselgel mit Trichloressigsäure/Toluol als mobile Phase).

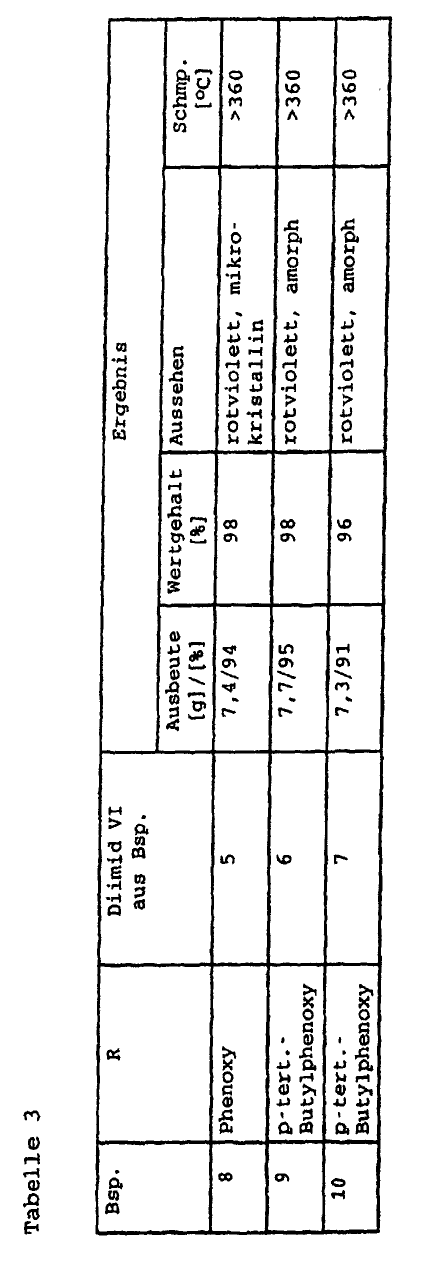

Analytische Daten zu Beispiel 8:
Elementaranalyse (Gew.-% ber./gef.):
C: 75,0/74,8; H: 2,8/2,8; O: 22,2/22,3;
IR (KBr): ν = 1758 (s, C=O), 1729 (s, C=O) cm⁻¹;
UV/VIS (H₂SO₄): λₘₐₓ (ε) = 415 (8832), 559 (38103) nm.

Analytische Daten zu Beispiel 9 und 10:
Elementaranalyse (Gew.-% ber./gef.):
C: 76,7/76,6; H: 4,7/4,7; O: 18,6/18,7;
IR (KBr): ν = 1755 (s, C=O), 1730 (s, C=O);
UV/VIS (H₂SO₄) : λₘₐₓ (*ε*) = 412 (9405), 561 (32746) nm.

### B2) Herstellung von 1,7-Di(p-tert.-butylphenoxy)perylen-3,4,9,10-tetracarbonsäuredianhydrid (I') nach der einstufigen Verfahrensvariante

### Beispiel 11

Zu einer Suspension von 10 g (18,2 mmol) 1,7-Dibromperylen-3,4,9,10-tetracarbonsäuredianhydrid (II) (Beispiel 1) in 320 ml N-Methylpyrrolidon wurden unter Rühren zunächst 8,3 g (60 mmol) wasserfreies Kaliumcarbonat und dann 13,65 g (91 mmol) p-tert.-Butylphenol zugegeben. Anschließend wurde das Reaktionsgemisch unter Stickstoff auf 120°C erhitzt und 6 h bei dieser Temperatur gerührt.

Nach dem Abkühlen auf 50°C wurde das ausgefallene Reaktionsprodukt abfiltriert, mit 50°C warmem N-Methylpyrrolidon gewaschen und dann kurz in 500 ml 6 gew.-%iger Salzsäure aufgerührt. Nach dem Abdekantieren der überstehenden Lösung wurde der verbleibende Feststoff mit 600 ml Wasser versetzt und unter Rühren 0,5 h auf 80-85°C erhitzt. Das Produkt wurde heiß abfiltriert, mit heißem Wasser neutral gewaschen und bei 120°C im Vakuum getrocknet.

Es wurden 8,6 g I' als rotviolettes Pulver mit einem Schmelzpunkt >360°C und einem Wertgehalt von 90 % erhalten, was einer Ausbeute von 63 % entspricht.

### Beispiel 11a

Zur Reinigung wurden 10 g nach Beispiel 11 erhaltenes 1,7-Di(p-tert.-butylphenoxy)perylen-3,4,9,10-tetracarbonsäuredianhydrid (I') 1 h in 2 l N-Methylpyrrolidon gerührt. Diese Mischung wurde anschließend über eine zu 2/3 mit Kieselgel gefüllte 21-G4-Glasfritte filtriert (N-Methylpyrrolidon als Eluens).

Aus dem Filtrat wurden nach verdampfen des N-Methylpyrrolidons im Vakuum 8,1 g I' als rotviolettes, kristallines Pulver mit einem Wertgehalt >98 % erhalten, dessen analytische Daten den zu Beispiel 9 angegebenen Daten entsprechen.

## Patentansprüche

1. 1,7-Disubstituierte Perylen-3,4,9,10-tetracarbonsäuredianhydride der allgemeinen Formel I und Perylen-3,4,9,10-tetracarbonsäuren der allgemeinen Formel Ia 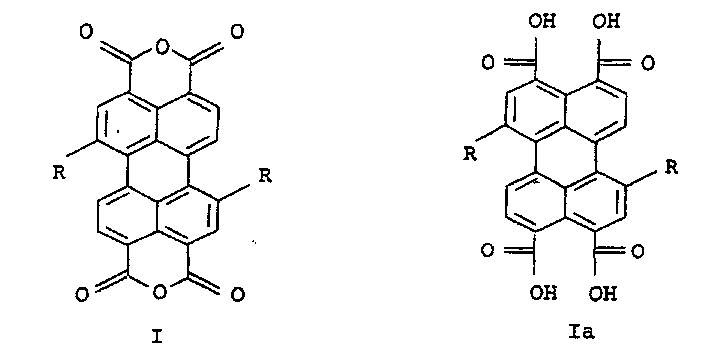 in der
R Aryloxy, Arylthio, Hetaryloxy oder Hetarylthio bedeutet, das jeweils durch C₁-C₃₀-Alkyl, dessen Kohlenstoffkette durch eine oder mehrere Gruppierungen -O-, -S-, -NR¹-, -CO- und/oder -SO₂- unterbrochen sein kann und/oder das durch -COOR¹, -SO₃R¹, Hydroxy, Cyano, C₁-C₆-Alkoxy, C₅-C₈-Cycloalkyl oder einen über ein Stickstoffatom gebundenen, 5- bis 7-gliedrigen heterocyclischen Rest, der weitere Heteroatome enthalten kann, ein- oder mehrfach substituiert sein kann, oder durch C₁-C₆-Alkoxy, Cyano, -COOR¹ oder -SO₃R¹ ein- oder mehrfach substituiert sein kann, wobei R¹ für Wasserstoff oder C₁-C₆-Alkyl steht.

2. Perylen-3,4,9,10-tetracarbonsäuredianhydride der Formel I und Perylen-3,4,9,10-tetracarbonsäuren der Formel Ia nach Anspruch 1, in der R Aryloxy oder Hetaryloxy bedeutet, das jeweils durch C₁-C₁₈-Alkyl, -COOR¹, -SO₃R¹ oder C₁-C₆-Alkoxy einoder mehrfach substituiert sein kann.

3. Verfahren zur Herstellung von 1,7-disubstituierten Perylen- 3,4,9,10 - tetracarbonsäuredianhydriden der Formel I bzw. den Perylen-3,4,9,10-tetracarbonsäuren der Formel Ia gemäß Anspruch 1 oder 2, **dadurch gekennzeichnet, daß** man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III
R² ― NH₂ III
in der R² C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann, bedeutet, umsetzt,
b) die in Schritt a) gebildeten 1,7-Dibromperylen-3,4,9,10-tetracarbonsäurediimide der allgemeinen Formel IV 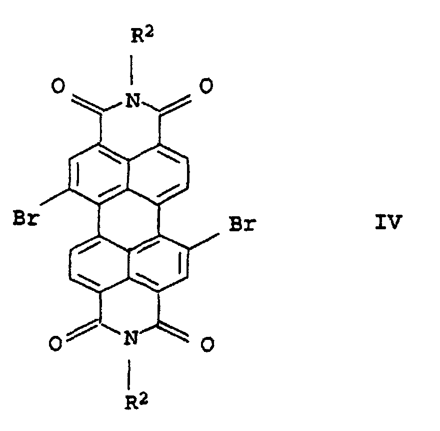 in Gegenwart eines inerten aprotischen Lösungsmittels und einer nicht oder nur schwach nucleophilen Base mit einem aromatischen Alkohol oder Thioalkohol der allgemeinen Formel V
H ― R V
umsetzt und
c) die in Schritt b) gebildeten, 1,7-disubstituierten Perylen-3,4,9,10-tetracarbonsäurediimide der allgemeinen Formel VI 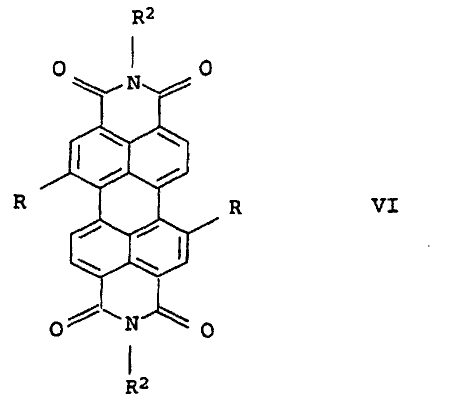 in Gegenwart eines polaren protischen Lösungsmittels und einer Base zu den 1,7-disubstituierten Perylen-3,4,9,10-tetracarbonsäuredianhydriden (I) bzw. den Perylen-3,4,9,10-tetracarbonsäuren (Ia) verseift.

4. 1,7-Disubstituierte Perylen-3,4,9,10-tetracarbonsäurediimide der allgemeinen Formel VI 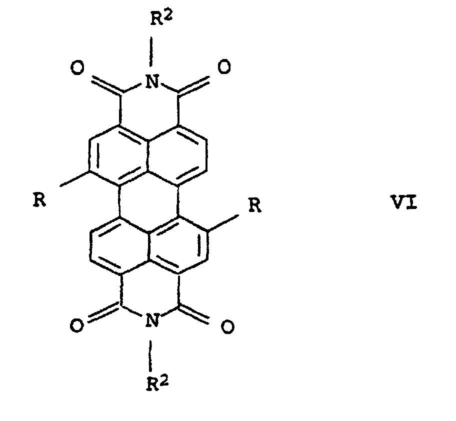 in der die Variablen folgende Bedeutung haben:
R Aryloxy, Arylthio, Hetaryloxy oder Hetarylthio, das jeweils durch C₁-C₃₀-Alkyl, dessen Kohlenstoffkette durch eine oder mehrere Gruppierungen -O-, -S-, -NR¹-, -CO- und/oder -SO₂- unterbrochen sein kann und/oder das durch -COOR¹, -SO₃R¹, Hydroxy, Cyano, C₁-C₆-Alkoxy, C₅-C₈-Cycloalkyl oder einen über ein Stickstoffatom gebundenen, 5-bis 7-gliedrigen heterocyclischen Rest, der weitere Heteroatome enthalten kann, ein- oder mehrfach substituiert sein kann, oder durch C₁-C₆-Alkoxy, Cyano, -COOR¹ oder -SO₃R¹ einfach substituiert sein kann, wobei R¹ für Wasserstoff oder C₁-C₆-Alkyl steht;
R² C₄-C₃₀-Alkyl, dessen Kohlenstoffkette durch eine oder mehrere Gruppierungen -O-, -S- oder -CO- unterbrochen sein kann, C₅-C₈-Cycloalkyl oder Aryl, das durch C₁-C₆-Alkyl oder C₁-C₆-Alkoxy ein- oder mehrfach substituiert sein kann.

5. Perylen-3,4,9,10-tetracarbonsäurediimide der allgemeinen Formel VI nach Anspruch 4, in der die Variablen folgende Bedeutung haben:
R Aryloxy oder Hetaryloxy, das jeweils durch C₁-C₁₈-Alkyl, -COOR¹, -SO₃R¹ oder C₁-C₆-Alkoxy ein- oder mehrfach substituiert sein kann;
R² C₅-C₈-Cycloalkyl oder Phenyl, das in den meta- und/oder para-Positionen durch C₁-C₆-Alkyl oder C₁-C₆-Alkoxy substituiert sein kann.

6. Verfahren zur Herstellung von 1,7-disubstituierten Perylen-3,4,9,10-tetracarbonsäurediimiden der Formel VI gemäß Anspruch 4 oder 5, **dadurch gekennzeichnet, daß** man
a) 1,7-Dibromperylen-3,4,9,10-tetracarbonsäuredianhydrid (II) bzw. 1,7-Dibromperylen-3,4,9,10-tetracarbonsäure (IIa) in Gegenwart eines polaren aprotischen Lösungsmittels und gegebenenfalls eines Imidierungskatalysators mit einem primären Amin der allgemeinen Formel III
R² ― NH₂ III
umsetzt und
b) die in Schritt a) gebildeten 1,7-Dibromperylen-3,4,9,10-tetracarbonsäurediimide der allgemeinen Formel IV 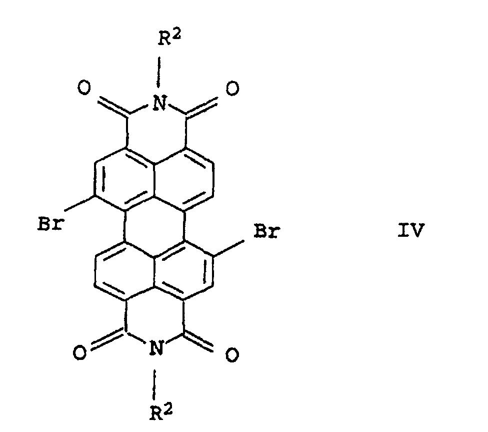 in Gegenwart eines inerten aprotischen Lösungsmittels und einer nicht oder nur schwach nucleophilen Base mit einem aromatischen Alkohol oder Thioalkohol der allgemeinen Formel V
H ― R V
umsetzt.

7. Verfahren zur Herstellung von 1,7-disubstituierten Perylen-3,4,9,10-tetracarbonsäuredianhydriden der Formel I bzw. den Perylen-3,4,9,10-tetracarbonsäuren der Formel Ia gemäß Anspruch 1 oder 2, **dadurch gekennzeichnet, daß** man 1,7-Dibromperylen-3,4,9,10-tetracarbonsäuredianhydrid (II) bzw. 1,7-Dibromperylen-3,4,9,10-tetracarbonsäure (IIa) in Gegenwart eines inerten aprotischen Lösungsmittels und einer nicht oder nur schwach nucleophilen Base mit einem aromatischen Alkohol oder Thioalkohol der allgemeinen Formel V
H ― R V
umsetzt.

8. Verfahren zur Herstellung von 1,7-Dibromperylen-3,4,9,10-tetracarbonsäuredianhydrid (II) bzw. 1,7-Dibromperylen-3,4,9,10-tetracarbonsäure (IIa) durch Bromierung von Perylen-3,4,9,10-tetracarbonsäuredianhydrid bzw. Perylen-3,4,9,10-tetracarbonsäure in 100 gew.-%iger Schwefelsäure, **dadurch gekennzeichnet, daß** man die Bromierung in Gegenwart von Iod bei einer Temperatur von 80 bis 90°C vornimmt und das Brom langsam zudosiert.

9. Verwendung von 1,7-disubstituierten Perylen-3,4,9,10-tetracarbonsäuredianhydriden der Formel I oder Perylen-3,4,9,10-tetracarbonsäuren der Formel Ia gemäß Anspruch 1 oder 2 als Pigmente, Laserfarbstoffe und Vorstufen für die Herstellung von Fluoreszenzfarbstoffen, polymeren Farbmitteln, Pigmenten und Pigmentadditiven.

10. Verwendung von 1,7-disubstituierten Perylen-3,4,9,10-tetracarbonsäurediimiden der Formel VI gemäß Anspruch 4 oder 5 als Pigmente und Farbstoffe zum Einfärben von hochmolekularen organischen und von anorganischen Materialien, als Laserfarbstoffe und organische Materialien für Elektrolumineszenzanwendungen.

## Claims

1. A 1,7-disubstituted perylene-3,4,9,10-tetracarboxylic dianhydride of the general formula I or perylene-3,4,9,10-tetracarboxylic acid of the general formula Ia 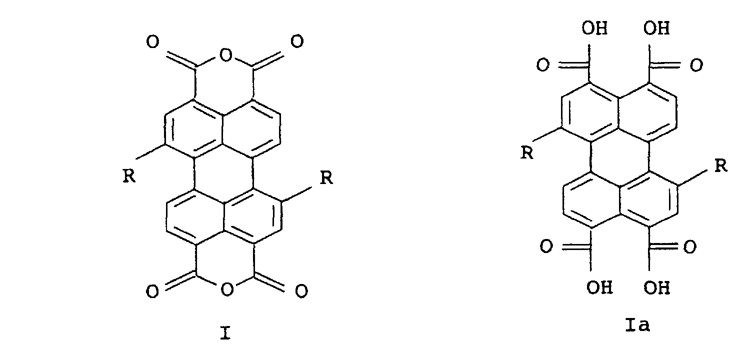 where
R is aryloxy, arylthio, hetaryloxy or hetarylthio each of which can be substituted one or more times by C₁-C₃₀-alkyl whose carbon chain can be interrupted by one or more groups -O-, -S-, -NR¹-, -CO- and/or -SO₂- and/or can be substituted one or more times by -COOR¹, -SO₃R¹, hydroxyl, cyano, C₁-C₆-alkoxy, C₅-C₈-cycloalkyl or a 5- to 7-membered heterocyclic radical which is attached via nitrogen and can contain further heteroatoms, or can be substituted one or more times by C₁-C₆-alkoxy, cyano, -COOR¹ or -SO₃R¹, where R¹ is hydrogen or C₁-C₆-alkyl.

2. A compound as claimed in claim 1, where R is aryloxy or hetaryloxy each of which can be substituted one or more times by C₁-C₁₈-alkyl, -COOR¹, -SO₃R¹ or C₁-C₆-alkoxy.

3. A process for the preparation of a compound as claimed in claim 1 or 2, which comprises
a) reacting 1,7-dibromoperylene-3,4,9,10-tetracarboxylic dianhydride (II) or 1,7-dibromoperylene-3,4,9,10-tetracarboxylic acid (IIa), in the presence of a polar aprotic solvent and in the presence or absence of an imidation catalyst, with a primary amine of the general formula III
R²― NH₂ III
in which R² is C₄-C₃₀-alkyl whose carbon chain can be interrupted by one or more groups -O-, -S- or -CO-, or is C₅-C₈-cycloalkyl or aryl which can be substituted one or more times by C₁-C₆-alkyl or C₁-C₆-alkoxy,
b) reacting the 1,7-dibromoperylene-3,4,9,10-tetracarboxylic diimides, formed in step a), of the general formula IV 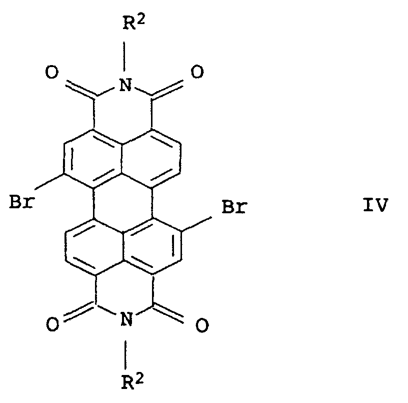 in the presence of an inert aprotic solvent and of a non-nucleophilic or only weakly nucleophilic base with an aromatic alcohol or thioalcohol of the general formula V
H ― R V
and
c) hydrolyzing the 1,7-disubstituted perylene-3,4,9,10-tetracarboxylic diimides, formed in step b), of the general formula VI 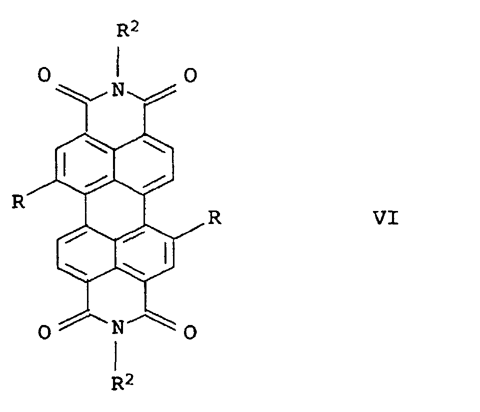 in the presence of a polar protic solvent and a base to give the compound (I) or (Ia).

4. A 1,7-disubstituted perylene-3,4,9,10-tetracarboxylic diimide of the general formula VI 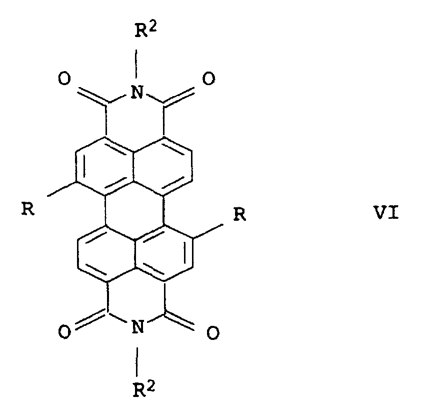 where
R is aryloxy, arylthio, hetaryloxy or hetarylthio each of which can be substituted one or more times by C₁-C₃₀-alkyl whose carbon chain can be interrupted by one or more groups -O-, -S-, -NR¹-, -CO- and/or -SO₂- and/or can be substituted one or more times by -COOR¹, -SO₃R¹, hydroxyl, cyano, C₁-C₆-alkkoxy, C₅-C₈-cycloalkyl or a 5- to 7-membered heterocyclic radical which is attached via nitrogen and can contain further heteroatoms, or can be substituted one or more times by C₁-C₆-alkoxy, cyano, -COOR¹ or -SO₃R¹, where R¹ is hydrogen or C₁-C₆-alkyl, and
R² is C₄-C₃₀-alkyl whose carbon chain can be interrupted by one or more groups -O-, -S- or -CO-, or is C₅-C₈-cycloalkyl or aryl which can be substituted one or more times by C₁-C₆-alkyl or C₁-C₆-alkoxy.

5. A compound of the general formula VI as set forth in claim 4, where:
R is aryloxy or hetaryloxy each of which can be substituted one or more times by C₁-C₁₈-alkyl, -COOR¹, -SO₃R¹ or C₁-C₆-alkoxy; and
R² is C₅-C₈-cycloalkyl or phenyl which can be substituted in the meta and/or para positions by C₁-C₆-alkyl or C₁-C₆-alkoxy.

6. A process for the preparation of a compound of the formula VI as claimed in claim 4 or 5, which comprises
a) reacting 1,7-dibromoperylene-3,4,9,10-tetracarboxylic dianhydride (II) or 1,7-dibromoperylene-3,4,9,10-tetracarboxylic acid (IIa), in the presence of a polar aprotic solvent and in the presence or absence of an imidation catalyst, with a primary amine of the general formula III
R²― NH₂ III
and
b) reacting the 1,7-dibromoperylene-3,4,9,10-tetracarboxylic diimides, formed in step a), of the general formula IV 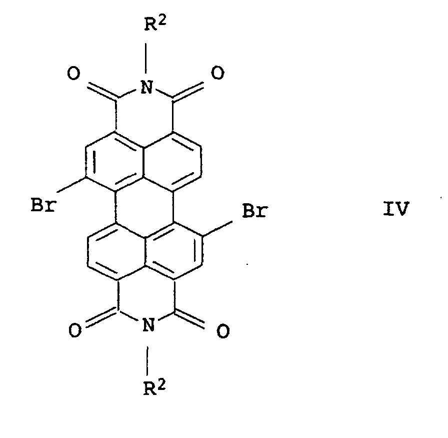 in the presence of an inert aprotic solvent and of a non-nucleophilic or only weakly nucleophilic base with an aromatic alcohol or thioalcohol of the general formula V
H ― R V.

7. A process for the preparation of a compound of the formula I or Ia as claimed in claim 1 or 2, which comprises reacting 1,7-dibromoperylene-3,4,9,10-tetracarboxylic dianhydride (II) or, respectively, 1,7-dibromoperylene-3,4,9,10-tetracarboxylic acid (IIa) in the presence of an inert aprotic solvent and of a non-nucleophilic or weakly nucleophilic base with an aromatic alcohol or thioalcohol of the general formula V
H ― R V.

8. A process for the preparation of 1,7-dibromoperylene-3,4,9,10-tetracarboxylic dianhydride (II) or 1,7-dibromoperylene-3,4,9,10-tetracarboxylic acid (IIa) by brominating perylene-3,4,9,10-tetracarboxylic dianhydride or, respectively, perylene-3,4,9,10-tetracarboxylic acid in 100% by weight sulfuric acid, which comprises brominating in the presence of iodine at 80-90°C and metering in the bromine slowly.

9. The use of a compound of the formula I or Ia as claimed in claim 1 or 2 as a pigment, laser dye or precursor for preparing fluorescent dyes, polymeric colorants, pigments and pigment additives.

10. The use of a compound of the formula VI as claimed in claim 4 or 5 as a pigment or dye for coloring high molecular mass organic materials and inorganic materials, as a laser dye or an organic material for electroluminescence applications.

## Revendications

1. Dianhydrides de l'acide pérylène-3,4,9,10-tétracarboxylique disubstitués en 1,7 de formule générale I et acides pérylène-3,4,9,10-tétracarboxyliques disubstitués en 1,7 de formule générale Ia 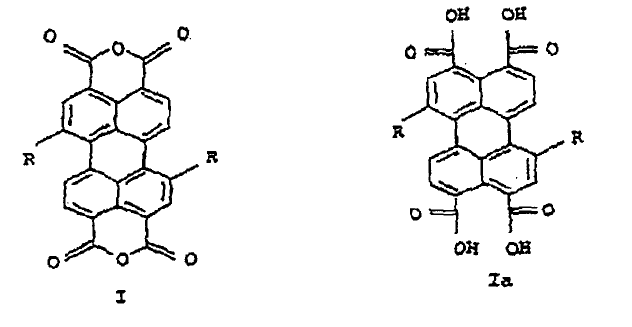 formules dans lesquelles
R représente un groupe aryloxy, arylthio, hétéroaryloxy ou hétéroarylthio, qui peut être à chaque fois substitué une ou plusieurs fois par un groupe alkyle en C₁ à C₃₀ dont la chaîne carbonée peut être interrompue par un ou plusieurs groupements -O-, -S-, -NR¹-, -CO- et/ou -SO₂-, et/ou qui peut être substitué par un groupe -COOR¹-, -SO₃R¹-, hydroxy, cyano, alcoxy en C₁ à C₆, cycloalkyle en C₅ à C₈ ou par un groupe hétérocyclique à 5 à 7 éléments lié par un atome d'azote et pouvant contenir d'autres hétéroatomes, ou qui peut être substitué une ou plusieurs fois par un groupe alcoxy en C₁ à C₆, cyano, -COOR¹- ou -SO₃R¹-, R¹ représentant un atome d'hydrogène ou un groupe alkyle en C₁ à C₆.

2. Dianhydrides de l'acide pérylène-3,4,9,10-tétracarboxylique de formule I et acides pérylène-3,4,9,10-tétracarboxyliques de formule Ia selon la revendication 1, dans lesquels R représente un groupe aryloxy ou hétéroaryloxy qui peut être à chaque fois substitué une ou plusieurs fois par un groupe alkyle en C₁ à C₁₈, -COOR¹-, -SO₃R¹- ou alcoxy en C₁ à C₆.

3. Procédé pour préparer les dianhydrides de l'acide pérylène-3,4,9,10-tétracarboxylique disubstitués en 1,7 de formule I ou les acides pérylène-3,4,9,10-tétracarboxyliques disubstitués en 1,7 de formule Ia selon la revendication 1 ou 2, **caractérisé en ce que** l'on
a) fait réagir le dianhydride de l'acide 1,7-dibromopérylène-3,4,9,10-tétracarboxylique (II) ou l'acide 1,7-dibromopérylène-3,4,9,10-tétracarboxylique (IIa), en présence d'un solvant aprotique polaire et éventuellement d'un catalyseur d'imidation, avec une amine primaire de formule générale III
R²-NH₂ III
dans laquelle R² représente un groupe alkyle en C₄ à C₃₀ dont la chaîne carbonée peut être interrompue par un ou plusieurs groupements -O-, -S- ou -CO-, cycloalkyle en C₅ à C₈ ou aryle, qui peut être substitué une ou plusieurs fois par un groupe alkyle en C₁ à C₆ ou alcoxy en C₁ à C₆,
b) on fait réagir les diimides de l'acide 1,7-dibromopérylène-3,4,9,10-tétracarboxylique formés dans l'étape a) de formule générale IV 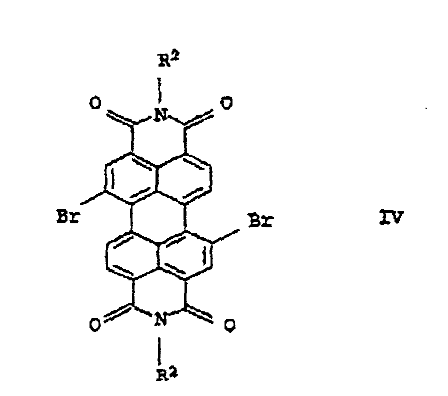 en présence d'un solvant aprotique inerte et d'une base non nucléophile ou seulement faiblement nucléophile, avec un alcool ou un thioalcool aromatique de formule générale V
H-R V
et **en ce que** l'on
c) saponifie les diimides de l'acide pérylène-3,4,9,10-tétracarboxylique disubstitués en 1,7 formés dans l'étape b) de formule générale VI 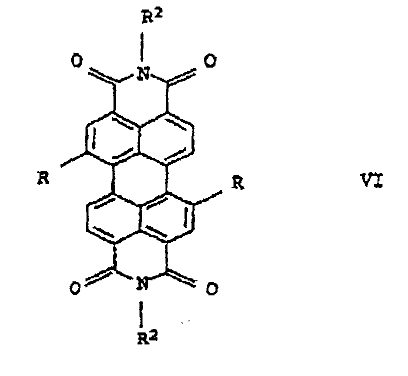 en présence d'un solvant protique polaire et d'une base, pour obtenir les dianhydrides de l'acide pérylène-3,4,9,10-tétracarboxylique disubstitués en 1,7 (I) ou les acides pérylène-3,4,9,10-tétracarboxyliques disubstitués en 1,7 (Ia).

4. Diimides de l'acide pérylène-3,4,9,10-tétracarboxylique disubstitués en 1,7 de formule générale VI 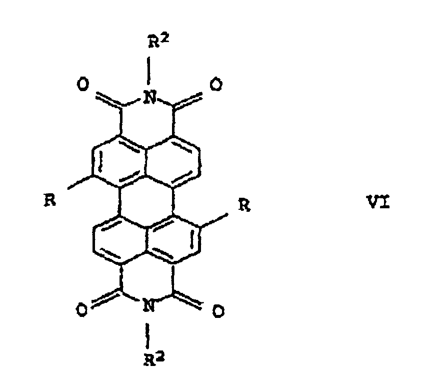 dans laquelle les variables prennent les significations suivantes:
R représente un groupe aryloxy, arylthio, hétéroaryloxy ou hétéroarylthio, qui peut être à chaque fois substitué une ou plusieurs fois par un groupe alkyle en C₁ à C₃₀ dont la chaîne carbonée peut être interrompue par un ou plusieurs groupements -O-, -S-, -NR¹-, -CO- et/ou -SO₂-, et/ou qui peut être substitué par un groupe -COOR¹-, -SO₃R¹-, hydroxy, cyano, alcoxy en C₁ à C₆, cycloalkyle en C₅ à C₈ ou par un groupe hétérocyclique à 5 à 7 éléments lié par un atome d'azote et pouvant contenir d'autres hétéroatomes, ou qui peut être substitué une fois par un groupe alcoxy en C₁ à C₆, cyano, -COOR¹- ou -SO₃R¹-, R¹ représentant un atome d'hydrogène ou un groupe alkyle en C₁ à C₆.
R² représente un groupe alkyle en C₄ à C₃₀ dont la chaîne carbonée peut être interrompue par un ou plusieurs groupements -O-, -S- ou -CO-, cycloalkyle en C₅ à C₈ ou aryle, qui peut être substitué une ou plusieurs fois par un groupe alkyle en C₁ à C₆ ou alcoxy en C₁ à C₆.

5. Diimides de l'acide pérylène-3,4,9,10-tétracarboxylique de formule générale VI selon la revendication 4, dans laquelle les variables prennent les significations suivantes:
R représente un groupe aryloxy ou hétéroaryloxy, qui peut être à chaque fois substitué une ou plusieurs fois par un groupe alkyle en C₁ à C₁₈, -COOR¹-, -SO₃R¹ ou alcoxy en C₁ à C₆;
R² représente un groupe cycloalkyle en C₅ à C₈ ou phényle, qui peut être substitué en position méta et/ou para par un groupe alkyle en C₁ à C₆ ou alcoxy en C₁ à C₆.

6. Procédé pour préparer les diimides de l'acide pérylène-3,4,9,10-tétracarboxylique disubstitués en 1,7 de formule VI selon la revendication 4 ou 5, **caractérisé en ce que** l'on
a) fait réagir le dianhydride de l'acide 1,7-dibromopérylène-3,4,9,10-tétracarboxylique (II) ou l'acide 1,7-dibromopérylène-3,4,9,10-tétracarboxylique (IIa), en présence d'un solvant aprotique polaire et éventuellement d'un catalyseur d'imidation, avec une amine primaire de formule générale III
R²-NH₂ III
et **en ce que** l'on
b) fait réagir les diimides de l'acide 1,7-dibromopérylène-3,4,9,10-tétracarboxylique formés dans l'étape a) de formule générale IV 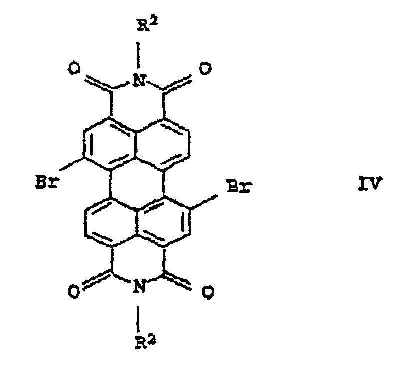 en présence d'un solvant aprotique inerte et d'une base non nucléophile ou seulement faiblement nucléophile, avec un alcool ou un thioalcool aromatique de formule générale V
H-R V

7. Procédé pour préparer les dianhydrides de l'acide pérylène-3,4,9,10-tétracarboxylique disubstitués en 1,7 de formule I ou les acides pérylène-3,4,9,10-tétracarboxyliques disubstitués en 1,7 de formule Ia selon la revendication 1 ou 2, **caractérisé en ce que** l'on fait réagir le dianhydride de l'acide 1,7-dibromopérylène-3,4,9,10-tétracarboxylique (II) ou l'acide 1,7-dibromopérylène-3,4,9,10-tétracarboxylique (IIa) en présence d'un solvant aprotique inerte et d'une base non nucléophile ou seulement faiblement nucléophile, avec un alcool ou un thioalcool aromatique de formule générale V
H-R V

8. Procédé pour préparer le dianhydride de l'acide 1,7-dibromopérylène-3,4,9,10-tétracarboxylique (II) ou l'acide 1,7-dibromopérylène-3,4,9,10-tétracarboxylique (IIa) au moyen de la bromation du dianhydride de l'acide pérylène-3,4,9,10-tétracarboxylique ou de l'acide pérylène-3,4,9,10-tétracarboxylique dans de l'acide sulfurique à 100%, **caractérisé en ce que** l'on réalise la bromation en présence d'iode, à une température allant de 80°C à 90°C, et **en ce que** l'on ajoute le brome lentement.

9. Utilisation des dianhydrides de l'acide pérylène-3,4,9,10-tétracarboxylique disubstitués en 1,7 de formule I ou des acides pérylène-3,4,9,10-tétracarboxyliques disubstitués en 1,7 de formule Ia selon la revendication 1 ou 2 en tant que pigments, colorants laser et précurseurs pour la préparation de colorants fluorescents, de colorants polymères, de pigments et d'additifs de pigments.

10. Utilisation des diimides de l'acide pérylène-3,4,9,10-tétracarboxylique disubstitués en 1,7 de formule VI selon la revendication 4 ou 5 en tant que pigments et colorants pour la coloration de matières organiques de masse moléculaire élevée et de matières inorganiques, en tant que colorants laser et matières organiques pour des applications électroluminescentes.
